# EUROPEAN PATENT APPLICATION

(11) **EP 4 036 230 A1**
(43) Date of publication of application: **03.08.2022**
(21) Application number: 20868713.7
(22) Date of filing: 25.09.2020
(51) Int. Cl.: C12N 15/09, C12N 9/78

(54) **OLIGONUCLEOTIDE, AND TARGET RNA SITE-SPECIFIC EDITING METHOD**

(30) Priority: 27.09.2019 JP 2019177118
(71) Applicant: Fukuoka University, Fukuoka-shi, Fukuoka 814-0180 (JP)
(72) Inventor: FUKUDA, Masatora, Fukuoka-shi, Fukuoka 814-0180 (JP)
(74) Representative: Plasseraud IP
(86) International application number: PCT/JP2020/036423
(87) International publication number: WO 2021/060527

(57) **Abstract**

Provided is an oligonucleotide that can induce editing activity specific to adenosine deaminase-1. The present invention is an oligonucleotide that includes a first oligonucleotide for identifying a target RNA and a second oligonucleotide linked to the 5' side of the first oligonucleotide, and that induces site-specific editing of the target RNA. The first oligonucleotide comprises: a target-corresponding nucleotide residue that corresponds to an adenosine residue in the target RNA; an oligonucleotide comprising 10-24 residues that is linked to the 3' side of the target-corresponding nucleotide residue and has a base sequence which is complementary to the target RNA; and an oligonucleotide comprising 3-6 residues that is linked to the 5' side of the target-corresponding nucleotide residue and has a base sequence which is complementary to the target RNA. The second oligonucleotide comprises 2-10 residues and forms a complementary strand to the target RNA, and the complementary strand includes at least one mismatched base.

### [Sequence list]

SEQUENCE LISTING

<110>  FUKUOKA UNIVERSITY

<120>  Oligonucleotide and method for site-directed  editing of  target
       RNA

<130>  675746

<160>  61

<170>  PatentIn version 3.5

<210>  1
<211>  910
<212>  RNA
<213>  Artificial Sequence

<220>
<223>  Rluc_WT

<400>  1
accauggcuu ccaaggugua cgaccccgag caacgcaaac gcaugaucac ugggccucag      60

uggugggcuc gcugcaagca aaugaacgug cuggacuccu ucaucaacua cuaugauucc     120

gagaagcacg ccgagaacgc cgugauuuuu cugcauggua acgcugccuc cagcuaccug     180

uggaggcacg ucgugccuca caucgagccc guggcuagau gcaucauccc ugaucugauc     240

ggaaugggua aguccggcaa gagcgggaau ggcucauauc gccuccugga ucacuacaag     300

uaccucaccg cuugguucga gcugcugaac cuuccaaaga aaaucaucuu ugugggccac     360

gacugggggg cuugucuggc cuuucacuac uccuacgagc accaagacaa gaucaaggcc     420

aucguccaug cugagagugu cguggacgug aucgaguccu gggacgagug gccugacauc     480

gaggaggaua ucgcccugau caagagcgaa gagggcgaga aaauggugcu ugagaauaac     540

uucuucgucg agaccaugcu cccaagcaag aucaugcgga aacuggagcc ugaggaguuc     600

gcugccuacc uggagccauu caaggagaag ggcgagguua gacggccuac ccucuccugg     660

ccucgcgaga ucccucucgu uaagggaggc aagcccgacg ucguccagau uguccgcaac     720

uacaacgccu accuucgggc cagcgacgau cugccuaaga uguucaucga guccgacccu     780

ggguucuuuu ccaacgcuau ugucgaggga gcuaagaagu ucccuaacac cgaguucgug     840

aaggugaagg gccuccacuu cagccaggag gacgcuccag augaaauggg uaaguacauc     900

aagagcuucg                                                            910


<210>  2
<211>  42
<212>  DNA
<213>  Artificial Sequence

<220>
<223>  primer

<400>  2
gaaggatcct tacaggtcct cctctgagat cagcttctgc tc                          42


<210>  3
<211>  33
<212>  DNA
<213>  Artificial Sequence

<220>
<223>  primer

<400>  3
gcagaattca ccatggcttc caaggtgtac gac                                    33


<210>  4
<211>  33
<212>  DNA
<213>  Artificial Sequence

<220>
<223>  primer

<400>  4
gacgctagca ccatggcttc caaggtgtac gac                                    33


<210>  5
<211>  21
<212>  DNA
<213>  Artificial Sequence

<220>
<223>  primer

<400>  5
cagctcgaac taagcggtga g                                                 21


<210>  6
<211>  42
<212>  DNA
<213>  Artificial Sequence

<220>
<223>  primer

<400>  6
gaactcgagt tacaggtcct cctctgagat cagcttctgc tc                          42


<210>  7
<211>  21
<212>  DNA
<213>  Artificial Sequence

<220>
<223>  primer

<400>  7
ctcaccgctt agttcgagct g                                                 21

<210>  8
<211>  39
<212>  DNA
<213>  Artificial Sequence

<220>
<223>  primer

<400>  8
ctaatacgac tcactatagg gctggactcc ttcatcaac                              39


<210>  9
<211>  19
<212>  DNA
<213>  Artificial Sequence

<220>
<223>  primer

<400>  9
ccagtcgtgg cccacaaag                                                    19


<210>  10
<211>  936
<212>  DNA
<213>  Artificial Sequence

<220>
<223>  Rluc_K41R

<400>  10
atggcttcca aggtgtacga ccccgagcaa cgcaaacgca tgatcactgg gcctcagtgg      60

tgggctcgct gcaagcaaat gaacgtgctg gactccttca tcaactacta tgattccgag     120

aggcacgccg agaacgccgt gatttttctg catggtaacg ctgcctccag ctacctgtgg     180

aggcacgtcg tgcctcacat cgagcccgtg gctagatgca tcatccctga tctgatcgga     240

atgggtaagt ccggcaagag cgggaatggc tcatatcgcc tcctggatca ctacaagtac     300

ctcaccgctt agttcgagct gctgaacctt ccaaagaaaa tcatctttgt gggccacgac     360

tgggggcctt gtctggcctt tcactactcc tacgagcacc aagacaagat caaggccatc     420

gtccatgctg agagtgtcgt ggacgtgatc gagtcctggg acgagtggcc tgacatcgag     480

gaggatatcg ccctgatcaa gagcgaagag ggcgagaaaa tggtgcttga gaataacttc     540

ttcgtcgaga ccatgctccc aagcaagatc atgcggaaac tggagcctga ggagttcgct     600

gcctacctgg agccattcaa ggagaagggc gaggttagac ggcctaccct ctcctggcct     660

cgcgagatcc ctctcgttaa gggaggcaag cccgacgtcg tccagattgt ccgcaactac     720

aacgcctacc ttcgggccag cgacgatctg cctaagatgt tcatcgagtc cgaccctggg     780

ttcttttcca acgctattgt cgagggagct aagaagttcc ctaacaccga gttcgtgaag     840

gtgaagggcc tccacttcag ccaggaggac gctccagatg aaatgggtaa gtacatcaag     900

agcttcgtgg agcgcgtgct gaagaacgag cagtaa                                936

<210>  11
<211>  279
<212>  RNA
<213>  Artificial Sequence

<220>
<223>  Rluc_sRNA

<400>  11
gggcuggacu ccuucaucaa cuacuaugau uccgagaggc acgccgagaa cgccgugauu      60

uuucugcaug guaacgcugc cuccagcuac cuguggaggc acgucgugcc ucacaucgag     120

cccguggcua gaugcaucau cccugaucug aucggaaugg guaaguccgg caagagcggg     180

aauggcucau aucgccuccu ggaucacuac aaguaccuca ccgcuuaguu cgagcugcug     240

aaccuuccaa agaaaaucau cuuugugggc cacgacugg                            279

<210>  12
<211>  27
<212>  RNA
<213>  Artificial Sequence

<220>
<223>  gRNA

<400>  12
cagcucaacc aagcggugag guacuug                                          27

<210>  13
<211>  24
<212>  RNA
<213>  Artificial Sequence

<220>
<223>  gRNA

<400>  13
cagcucaacc aagcggugag guac                                             24

<210>  14
<211>  27
<212>  RNA
<213>  Artificial Sequence

<220>
<223>  gRNA

<400>  14
cagcagcuca accaagcggu gagguac                                          27

<210>  15
<211>  70
<212>  RNA

<213>  Artificial Sequence

<220>
<223>  gRNA

<400>  15
gguucagcag cucgaaccaa gggguggaau aguauaacaa uaugcuaaau guuguuauag      60

uaucccaccu                                                              70


<210>  16
<211>  69
<212>  RNA
<213>  Artificial Sequence

<220>
<223>  gRNA

<400>  16
ggguggaaua guauaacaau augcuaaaug uuguuauagu aucccaccua accaagcggu      60

gagguacuu                                                               69


<210>  17
<211>  21
<212>  RNA
<213>  Artificial Sequence

<220>
<223>  ASR

<400>  17
gguucagcag cucgaaccaa g                                                 21


<210>  18
<211>  22
<212>  RNA
<213>  Artificial Sequence

<220>
<223>  ASR

<400>  18
ggaaccaagc ggugagguac uu                                                22


<210>  19
<211>  19
<212>  DNA
<213>  Artificial Sequence

<220>
<223>  primer

<400>  19
gctggactcc ttcatcaac                                                    19


<210>  20
<211>  21

<212>  RNA
<213>  Artificial Sequence

<220>
<223>  gRNA

<400>  20
cucaaccaag cggugaggua c                                                21


<210>  21
<211>  25
<212>  RNA
<213>  Artificial Sequence

<220>
<223>  gRNA

<400>  21
gcagcucaac caagcgguga gguac                                            25


<210>  22
<211>  27
<212>  RNA
<213>  Artificial Sequence

<220>
<223>  gRNA

<400>  22
cagcagcuca accaagcggu gagguac                                          27


<210>  23
<211>  30
<212>  RNA
<213>  Artificial Sequence

<220>
<223>  gRNA

<400>  23
guucagcagc ucaaccaagc ggugagguac                                       30


<210>  24
<211>  25
<212>  RNA
<213>  Artificial Sequence

<220>
<223>  gRNA

<400>  24
cagcucgaac caagcgguga gguac                                            25


<210>  25
<211>  27
<212>  DNA
<213>  Artificial Sequence

<220>
<223>  primer

<400>  25
gctctagaac catgggggt tctcatc                                          27


<210>  26
<211>  38
<212>  DNA
<213>  Artificial Sequence

<220>
<223>  primer

<400>  26
ctgagatcag cttctgctct actgggcaga gataaaag                             38


<210>  27
<211>  42
<212>  DNA
<213>  Artificial Sequence

<220>
<223>  primer

<400>  27
cgtaagcttc tacaggtcct cctctgagat cagcttctgc tc                        42


<210>  28
<211>  22
<212>  DNA
<213>  Artificial Sequence

<220>
<223>  primer

<400>  28
ctaatacgac tccactatag gg                                              22


<210>  29
<211>  18
<212>  DNA
<213>  Artificial Sequence

<220>
<223>  primer

<400>  29
tagaaggcac agtcgagg                                                   18


<210>  30
<211>  20
<212>  DNA
<213>  Artificial Sequence

<220>
<223>  primer

<400>  30
gcagcaccag gtgagtttcg                                                   20


<210>  31
<211>  21
<212>  DNA
<213>  Artificial Sequence

<220>
<223>  primer

<400>  31
gctcgtgaga tacctgaaca c                                                 21


<210>  32
<211>  20
<212>  DNA
<213>  Artificial Sequence

<220>
<223>  primer

<400>  32
gctccgtgtg gagatggcgc                                                   20


<210>  33
<211>  37
<212>  DNA
<213>  Artificial Sequence

<220>
<223>  primer

<400>  33
ggccacgcgt cgactagtac tttttttttt ttttttt                                37


<210>  34
<211>  42
<212>  DNA
<213>  Artificial Sequence

<220>
<223>  primer

<400>  34
ctaatacgac tcactatagg gaccatggct tccaaggtgt ac                          42


<210>  35
<211>  20
<212>  DNA
<213>  Artificial Sequence

<220>
<223>  primer

<400>  35
ggccacgcgt cgactagtac                                                   20

<210>  36
<211>  22
<212>  DNA
<213>  Artificial Sequence

<220>
<223>  primer

<400>  36
ttactgctcg ttcttcagca cg                                                22


<210>  37
<211>  30
<212>  DNA
<213>  Artificial Sequence

<220>
<223>  gRNA

<400>  37
cagctcaacc aagcggtgag gtacttgtag                                        30


<210>  38
<211>  33
<212>  DNA
<213>  Artificial Sequence

<220>
<223>  gRNA

<400>  38
cagctcaacc aagcggtgag gtacttgtag tga                                    33


<210>  39
<211>  160
<212>  RNA
<213>  Artificial Sequence

<220>
<223>  model target RNA

<400>  39
gggugaaugg ccacaaguuc agcgugagcg gcgagggcga gggcgaugcc accuacggca      60

agcugacccu gaaguucauc ugcaccaccg gcaagcugcc ugugcccugg cccacccugg     120

ugaccacccu gagcuacggc gugcagugcu ucucacgcua                           160


<210>  40
<211>  24
<212>  RNA
<213>  Artificial Sequence

<220>
<223>  gRNA

<400>  40
cugcacccgc agcucagggu gguc                                              24

<210>  41
<211>  223
<212>  RNA
<213>  Artificial Sequence

<220>
<223>  model target RNA

<400>  41
ggcugaugag agcaucggcc ugcaguugcc cuucagcagc ugguacgugg aucggggcgg      60

aaacggcugu cugauggccc cagagguguc cacggcccgu ccuggcccca gggcagugau      120

ugacuacagc aaggcugaug ccuaggcagu gggagccauc gccuaugaaa ucuucgggcu      180

ugucaauccc uucuacggcc agggcaaggc ccaccuugaa agc                        223


<210>  42
<211>  24
<212>  RNA
<213>  Artificial Sequence

<220>
<223>  gRNA

<400>  42
ucccacgccc aggcaucagc cuug                                              24


<210>  43
<211>  183
<212>  RNA
<213>  Artificial Sequence

<220>
<223>  model target RNA

<400>  43
ggaugaccuu cgucgcuuau gcauacucag gaugaguuuu gugaaaggcu ggggaccgga      60

uuacccaaga cagagcauca aagaaacacc uugcuggauu gaaauucacu uacaccgggc      120

ccuccagcuc cuagacgaag uacuucauac caugccgauu gcagacccac aaccuuuaga      180

cug                                                                     183


<210>  44
<211>  24
<212>  RNA
<213>  Artificial Sequence

<220>
<223>  gRNA

<400>  44
guacuugucc aggagcugga gggc                                              24


<210>  45

<211>  250
<212>  RNA
<213>  Artificial Sequence

<220>
<223>  model target RNA

<400>  45
ggacuuugau ugcacauugu uguuuuuuua auagucauuc caaauaugag augcguuguu      60

acaggaaguc ccuugccauc cuaaaagcca ccccacuucu cucuaaggag aauggcccag      120

uccucuccca aguccacaca ggggagguga uagcauugcu uucguguaaa uuauguaaug      180

caaaauuuuu uuaaucuucg ccuuaauacu uuuuuauuuu guuuuauuuu gaaugaugag      240

ccuucgugcc                                                             250


<210>  46
<211>  24
<212>  RNA
<213>  Artificial Sequence

<220>
<223>  gRNA

<400>  46
aaagcaugcc aucaccuccc cugu                                             24


<210>  47
<211>  24
<212>  RNA
<213>  Artificial Sequence

<220>
<223>  gRNA

<400>  47
cuuguaugac ccaggaggcg auau                                             24


<210>  48
<211>  24
<212>  RNA
<213>  Artificial Sequence

<220>
<223>  gRNA

<400>  48
cuuguaugac gcaggaggcg auau                                             24


<210>  49
<211>  24
<212>  RNA
<213>  Artificial Sequence

<220>
<223>  gRNA

<400>  49
acgccgagcc cagggugguc acca                                              24


<210>  50
<211>  24
<212>  RNA
<213>  Artificial Sequence

<220>
<223>  gRNA

<400>  50
acgccgagcc gagggugguc acca                                              24


<210>  51
<211>  24
<212>  RNA
<213>  Artificial Sequence

<220>
<223>  gRNA

<400>  51
aggcgaggcc cccacugccu aggc                                              24


<210>  52
<211>  24
<212>  RNA
<213>  Artificial Sequence

<220>
<223>  gRNA

<400>  52
aggcgaggcc gccacugccu aggc                                              24


<210>  53
<211>  24
<212>  RNA
<213>  Artificial Sequence

<220>
<223>  gRNA

<400>  53
augaagacuc cgucuaggag cugg                                              24


<210>  54
<211>  24
<212>  RNA
<213>  Artificial Sequence

<220>
<223>  gRNA

<400>  54
augaagacuc ggucuaggag cugg                                              24

<210>  55
<211>  28
<212>  RNA
<213>  Artificial Sequence

<220>
<223>  model target RNA

<400>  55
caaguaccuc accgcuuagu ucgagcug                                          28


<210>  56
<211>  25
<212>  RNA
<213>  Artificial Sequence

<220>
<223>  model target RNA

<400>  56
guaccucacc gcuuaguucg agcug                                             25


<210>  57
<211>  25
<212>  RNA
<213>  Artificial Sequence

<220>
<223>  model target RNA


<220>
<221>  misc_feature
<222>  (19)..(19)
<223>  n is a, c, g, or u

<400>  57
guaccucacc gcuuaguung agcug                                             25


<210>  58
<211>  25
<212>  RNA
<213>  Artificial Sequence

<220>
<223>  model target RNA

<400>  58
guaccucacc gcuuaguucg agcug                                             25


<210>  59
<211>  31
<212>  RNA
<213>  Artificial Sequence

<220>
<223>  model target RNA

<400>  59
cuacaaguac cucaccgcuu aguucgagcu g                                    31


<210>  60
<211>  34
<212>  RNA
<213>  Artificial Sequence

<220>
<223>  model target RNA

<400>  60
ucacuacaag uaccucaccg cuuaguucga gcug                                 34


<210>  61
<211>  25
<212>  RNA
<213>  Artificial Sequence

<220>
<223>  model target RNA

<400>  61
auaucgccuc cuggaucacu acaag                                           25

## Description

### Technical Field

The present invention relates to methods for site-specific editing of oligonucleotides and target RNAs.

### Background Art

With the development of genome editing technology, the method of controlling biological phenomena by modifying the genetic information which is the blueprint of living organisms, namely the DNA information in cells, is beginning to be used in the fields of medical and drug discovery as an approach to treat diseases. Since DNA is a permanent and unchanging molecule in the cells, the effect of DNA modification remains permanently in the subject cell or subject organism. On the other hand, RNA is a transient genetic information molecule. Therefore, modification of RNA information can confer a temporary non-permanent genetic information modification effect in the target organism.

As an RNA modification technique, for example, International Publication No. 2016/097212 describes oligonucleotide constructs for site-specific editing of nucleotides in a target RNA sequence, which include a target-directed moiety containing an antisense sequence complementary to a part of the target RNA, and a recruitment moiety that can attach to and recruit an RNA editing entity that can edit nucleotides which are present in the cells. Further, for example, International Publication No. 2017/010556 describes a method for introducing site-specific RNA mutations in which double-strand specific adenosine deaminase (ADAR) is allowed to act on a complex of a target RNA and a target editing guide RNA.

As an adenosine deaminase having RNA editing activity, ADAR1 and ADAR2 isoforms are known, and these are considered to be expressed in the living body in a cell-type-specific manner. For example, ADAR1 is said to be rarely expressed in human skeletal muscle cells. On the other hand, ADAR2 is said to be hardly expressed in human bone marrow cells. In addition, it is generally considered that ADAR1 is more expressed in human cells.

### Summary of the Invention

### Problems to be Solved by the Invention

If the ADAR1 editing activity can be induced in an isoform-specific manner, it will be possible to edit RNAs specifically by cell type. It is also believed that RNA editing can be made possible in human cells more efficiently. Therefore, an objective of the present invention is to provide oligonucleotides that are capable of inducing ADAR1-specific editing activity.

### Means for Solving the Problems

Specific means for solving the above problems are as follows, and the present invention encompasses the aspects below. The first aspect is an oligonucleotide comprising a first oligonucleotide that identifies the target RNA and a second oligonucleotide linked to the 5' side of the first oligonucleotide to induce site-specific editing of the target RNA. The first oligonucleotide is composed of a target-corresponding nucleotide residue that corresponds to an adenosine residue in the target RNA, a 10 to 24 residue oligonucleotide linked to the 3' side of the target-corresponding nucleotide residue and having a base sequence complementary to the target RNA, and a 3 to 6 residue oligonucleotide linked to the 5' side of the target-corresponding nucleotide residue and having a base sequence complementary to the target RNA. The second oligonucleotide has 2 to 10 residues and forms a complementary strand with the target RNA. The complementary strand formed by the second oligonucleotide contains at least one mismatched base.

The second aspect is a method for site-specific editing of target RNA, which comprises contacting the oligonucleotide with the target RNA in the presence of adenosine deaminase 1.

According to the present invention, it is possible to provide an oligonucleotide capable of inducing ADAR1-specific editing activity.

### Brief Description of the Drawings

[Figure 1] Figure 1 is a schematic diagram showing a complex consisting of a target RNA and a target editing guide RNA.
[Figure 2] Figure 2 is a diagram showing the results of editing and analyzing a target RNA *in vitro.*
[Figure 3] Figure 3 is a schematic diagram showing a complex consisting of a target RNA and a target editing guide RNA.
[Figure 4] Figure 4 is a diagram showing the results of editing and analyzing a target RNA *in vitro.*
[Figure 5A] Figure 5A is a schematic diagram showing a complex consisting of a target RNA and a target editing guide RNA.
[Figure 5B] Figure 5B is a schematic diagram showing a complex consisting of a target RNA and a target editing guide RNA.
[Figure 6] Figure 6 is a diagram showing the results of editing and analyzing a target RNA *in vitro.*
[Figure 7] Figure 7 is a diagram showing the results of editing and analyzing a target RNA in cultured cells.
[Figure 8] Figure 8 is a diagram showing changes in the luminescence intensity due to target RNA editing in cultured cells.
[Figure 9] Figure 9 is a diagram showing the results of editing and analyzing a target RNA in cultured cells.
[Figure 10] Figure 10 is a diagram showing changes in the luminescence intensity due to target RNA editing in cultured cells.
[Figure 11A] Figure 11A is a schematic diagram showing a complex consisting of a target RNA and a target editing guide RNA.
[Figure 11B] Figure 11B is a schematic diagram showing a complex consisting of a target RNA and a target editing guide RNA.
[Figure 12] Figure 12 is a diagram showing the results of editing and analyzing various target RNAs *in vitro.*
[Figure 13A] Figure 13A is a schematic diagram showing a complex consisting of a target RNA and a target editing guide RNA.
[Figure 13B] Figure 13B is a schematic diagram showing a complex consisting of a target RNA and a target editing guide RNA.
[Figure 14] Figure 14 is a diagram showing the results of editing and analyzing a target RNA *in vitro.*
[Figure 15] Figure 15 is a diagram showing the results of editing and analyzing a target RNA *in vitro.*
[Figure 16] Figure 16 is a diagram showing the results of editing and analyzing a target RNA *in vitro.*

### Mode for Carrying Out the Invention

Hereinafter, embodiments of the present invention will be described in detail. However, the embodiments shown below illustrate oligonucleotides to embody the technical concept of the present invention, and the present invention is not limited to the oligonucleotides shown below.

### Target editing guide RNA

The oligonucleotide that induces site-specific editing of a target RNA (hereinafter, also referred to as a target editing guide RNA) includes a first oligonucleotide that identifies the target RNA and a second oligonucleotide that is linked to the 5' side of the first oligonucleotide. The first oligonucleotide is composed of a target-corresponding nucleotide residue that corresponds to an adenosine residue in the target RNA, a 10 to 24 residue oligonucleotide linked to the 3' side of the target-corresponding nucleotide residue and having a base sequence complementary to the target RNA, and a 3 to 6 residue oligonucleotide linked to the 5' side of the target-corresponding nucleotide residue and having a base sequence complementary to the target RNA. The second oligonucleotide has 2 to 10 residues and forms a complementary strand with the target RNA, and the complementary strand contains at least one mismatched base.

An oligonucleotide containing a short-chain second oligonucleotide capable of forming a complementary strand containing at least one mismatched base with the target RNA on the 5' side of the first oligonucleotide that identifies the target RNA can induce the editing activity of ADAR1 specifically. It can be considered that this is because, for example, the presence of the mismatched base works favorably for the recruitment of ADAR1 but inhibits the recruitment of ADAR2.

The target editing guide RNA of the present embodiment is shorter than the conventional-type target editing guide RNA, so that the manufacturing cost is simply reduced. Further, in addition to the method of introducing the target editing guide RNA into cells by gene expression, as chemical synthesis becomes easy, the method of directly introducing the chemically synthesized target editing guide RNA into cells is made easy. Along with this, existing artificial nucleic acids and such used in nucleic acid drug development and the like can be applied to the target editing guide RNA. This makes it possible to provide a more highly functional target editing guide RNA by improving intracellular degradation resistance, improving cell introduction efficiency, and the like. Furthermore, the target editing guide RNA is composed of the minimum number of residues required for induction of editing, thereby suppressing off-target editing at positions other than the adenosine residue, which is the target of editing, while maintaining specificity for the target RNA.

The target editing guide RNA induces site-specific editing for the target RNA, for example, by specifically recruiting ADAR1 among the ADARs that catalyze target editing to the target RNA. ADAR is an enzyme that converts adenosine residues in double-stranded RNA into inosine residues by a hydrolytic deamination reaction, and is widely present in mammalian cells. Since the inosine residue is similar in structure to the guanosine residue, it is translated as a guanosine residue at translation of the RNA information, and as a result, the RNA information is edited. When such RNA editing occurs in the portion encoding the amino acid, amino acid substitution or the like occurs even though there is no DNA mutation on the genome, and the function of the target gene can be controlled.

When introduced into mammalian cells, the target editing guide RNA can recruit ADAR1 present in the cells specifically to the target RNA to induce site-specific editing of the target RNA.

The target editing guide RNA of the present embodiment can induce ADAR1-specific editing activity. ADAR1 specificity means that, for example, the ratio of the editing-inducing activity to ADAR1 to the editing-inducing activity to ADAR2 *in vitro* is more than 1, and is preferably 1.5 or more, 2 or more, or 5 or more. In addition, the editing-inducing activity for ADAR1 and ADAR2 is evaluated under the condition that, when an editing reaction is performed on the HTR2C RNA (serotonin 2C receptor mRNA precursor), which is known to be edited by adenosine deaminase, the concentrations of ADAR1 and ADAR2 are adjusted so that the A site shows an editing ratio of about 30% for ADAR1 and the D site shows an editing ratio of about 90% for ADAR2. In addition, in cells, when the ADAR1 expression plasmid and the ADAR2 expression plasmid constructed using the same expression vector are used and expressed by transfection into cells under the same conditions, the editing is ADAR1 specific if ADAR1 has a higher efficiency of inducing editing at the same target editing site.

The first oligonucleotide contained in the target editing guide RNA identifies the target RNA. The target RNA is not particularly limited as long as it contains an adenosine residue to be edited, and may be either cellular RNA or viral RNA, and is usually an mRNA precursor or mRNA encoding a protein. The editing site in the target RNA may be in an untranslated region, a splice region, an exon, an intron, or any region that affects the stability, structure, or function of the RNA. The target RNA may also contain mutations to be modified or altered. Alternatively, the target RNA may be one whose sequence has been mutated to encode a phenotype different from the native form.

The target RNA is preferably an RNA that encodes a protein, and specifically the encoded protein is, for example, a phosphorylated protein involved in the signal transduction of serotonin receptors, glutamate receptors, membrane potential-dependent potassium channels, STAT3, NFkBIA, MAPK14, and the like.

Target editing guide RNA can be applied, for example, to the treatment of hereditary diseases. Hereditary diseases include cystic fibrosis, leukoderma, α-1 antitrypsin deficiency, Alzheimer's disease, muscular atrophic lateral sclerosis, asthma, β-thalassemia, CADASIL syndrome, Charcot-Marie-Tooth disease, chronic obstructive lung disease (COPD), distal spinal muscle atrophy (DSMA), Duchenne/Becker muscular dystrophy, dystrophic Epidermolysis bullosa, Epidermylosis bullosa, Fabry disease, Factor V Leiden-related disorders, familial adenomas polyposis, galactosemia, Gaucher disease, glucose-6-phosphate dehydrogenase deficiency, hemophilia, hereditary hemochromatosis, Hunter syndrome, Huntington's disease, Hurler syndrome, inflammatory bowel disease (IBD), hereditary polyagglutination syndrome, Leber's congenital amaurosis, Lesch-Nyhan syndrome, Lynch syndrome, Marfan syndrome, Mucopolysaccharidosis, muscular dystrophy, Myotonic dystrophy types I and II, Neurofibromatosis, Niemann-Pick disease types A, B and C, NY-esol-related pancreatic cancer, Parkinson's disease, Peutz-Jeghers syndrome, phenylketonuria, Pompe disease, Primary ciliary dyskinesia, prothrombin mutation-related diseases such as prothrombin G20210A mutation, pulmonary hypertension, retinitis pigmentosa, Sandhoff disease, severe combined immunodeficiency syndrome (SCID), sickle cell anemia, spinal muscle atrophy, Stargardt disease, Tay-Sachs disease, Usher syndrome, X-linked immunodeficiency, and various forms of cancer (for example, BRCA1 and 2 related breast cancer, ovarian cancer, etc.).

The first oligonucleotide consists of a target-corresponding nucleotide residue that corresponds to an adenosine residue to be edited in the target RNA, and an oligonucleotide of 10 to 24 residues the 3' side of the target-corresponding nucleotide residue and having a complementary base sequence to the 5' side of the target-corresponding nucleotide residue, and a 3' side oligonucleotide of 3 to 6 resides having a base sequence complementary to the corresponding base sequence of the target RNA. Oligonucleotides linked to the 5' side and 3' side of the target-corresponding nucleotide residue form a double strand with the target RNA to form a complementary strand (hereinafter, also referred to as the first complementary strand) as a whole, and the target RNA and the target sites to be edited in the target RNA are identified. Here, the complementary base sequence contains, for example, a base sequence forming thermodynamically stable non-Watson-Crick type wobble base pairs such as G-U base pairs, in addition to the base sequence forming the Watson-Crick type base pairs.

The target-corresponding nucleotide residue is a nucleotide residue that corresponds to an adenosine residue to be edited, and is, for example, a cytidine residue, a uridine residue, an adenosine residue or a derivative thereof. The target-corresponding nucleotide residue is preferably a residue that does not form a base pair with the adenosine residue to be edited, more preferably a cytidine residue or a derivative thereof, and even more preferably a cytidine residue.

In the first oligonucleotide, the number of residues of the oligonucleotide linked to the 3' side of the target-corresponding nucleotide residue and having a base sequence complementary to the target RNA is 10 or more and 24 or less, preferably 11 or more, 12 or more, or 13 or more, and more preferably 22 or less, 20 or less, 18 or less, 16 or less or 15 or less. When the number of residues is in the above range, the specificity for ADAR1 tends to be further improved.

In the first oligonucleotide, the oligonucleotide linked to the 3' side of the target-corresponding nucleotide residue may have a complementary base sequence that does not contain a mismatched base with respect to the target RNA. In that case, it is preferable that in the target RNA, the guanosine residue is not linked to the 5' side of the adenosine residue to be edited. That is, when the nucleotide linked to the 5' side of the adenosine residue which is the editing target of the target RNA is an adenosine residue, a cytidine residue, or a uridine residue, it is preferable that in the first oligonucleotide, the oligonucleotide linked to the 3' side of the target-corresponding nucleotide residue may have a complementary base sequence that does not contain a mismatched base with respect to the target RNA.

In the first oligonucleotide, the oligonucleotide linked to the 3' side of the target-corresponding nucleotide residue may contain a base that is non-complementary to the base sequence of the target RNA. For example, in a target RNA in which a guanosine residue is linked to the 5' side of the adenosine residue to be edited, the editing-inducing activity may decrease. Even in that case, the editing-inducing activity can be improved by a first oligonucleotide having a base sequence containing a base non-complementary to the guanosine residue. The non-complementary base to the guanosine residue may be, for example, a guanosine residue. That is, in the target editing guide RNA for which the guanosine residue is linked to the 5' side of the adenosine residue to be edited, the guanosine residue may be linked to the 3' side of the target-corresponding nucleotide residue.

In the first oligonucleotide, the number of residues of the oligonucleotide linked to the 5' side of the target-corresponding nucleotide residue and having a base sequence complementary to the target RNA is 3 or more and 6 or less, and is preferably 3 or more and 5 or less, 3 or more and 4 or less, or 3. When the number of residues is within the above range, the specificity for ADAR1 tends to be further improved.

The second oligonucleotide forms a complementary strand (hereinafter, also referred to as a second complementary strand) containing one or more mismatched bases with the target RNA. The number of residues of the second oligonucleotide is 2 or more and 10 or less, or 4 or more and 8 or less. The number of residues of the second oligonucleotide is, for example, 3 or more, preferably 4 or more, or 5 or more, and for example, 10 or less, preferably 9 or less, 8 or less, or 7 or less. When the number of residues is within the above range, induction of the editing tends to be further enhanced. The number of mismatched bases in the second complementary strand is, for example, 3 or less, and is preferably 1.

The mismatched base in the second complementary strand may be one that forms a mismatched base pair, and may be derived from a nucleotide residue inserted into at least one of the target RNA or the second oligonucleotide that forms the fully complementary strand. The mismatched base is preferably derived from a nucleotide residue inserted into the fully complementary strand, and more preferably derived from a nucleotide residue inserted into the target RNA side of the fully complementary strand. This tends to further enhance the induction of editing. Here, the mismatched base pair means that the corresponding two bases in the target RNA and the second oligonucleotide are a combination that does not form a stable base pair.

The position of the mismatched base in the second complementary strand is preferably any of the first to third residues on the 3' side of the target RNA of the first complementary strand consisting of the target RNA and the first oligonucleotide, or on the 5' side of the first oligonucleotide, and it is more preferable that it is the first residue.

The mismatched base in the second complementary strand may be, for example, derived from the nucleotide residue inserted in the first residue linked to the 3' side of the target RNA forming the first complementary strand, and may be a mismatched base pair formed at the first residue on the 5' side of the first oligonucleotide forming the first complementary strand.

In one aspect of the target editing guide RNA, the first oligonucleotide forming the first complementary strand with the target RNA has a cytidine residue as a target-corresponding nucleotide residue and a base sequence complementary to the base sequence of the target RNA, an oligonucleotide having 12 to 16 residues linked to the 3' side of the target-corresponding nucleotide residue and a base sequence complementary to the base sequence of the target RNA, and an oligonucleotide having 3 residues linked to the 5' side of the target-corresponding nucleotide residue; and the second oligonucleotide has a base sequence complementary to the target RNA forming the first complementary strand after the second residue on the 3' side and comprises an oligonucleotides with 4 to 8 residues.

The nucleotide residues constituting the target editing guide RNA may be a natural ribonucleotide residue or a non-natural modified nucleotide residue. Modified nucleotide residues include those modified with a phosphodiester bond between nucleosides, those modified with a 2' hydroxyl group of ribose, those containing an intramolecularly cross-linked ribose, and those modified with at least one of a purine base and a pyrimidine base. Examples of modification of the phosphodiester bond moiety include phosphorothioation, methylphosphonation, methylthiophosphonation, phosphorodithioation, phosphoramidation, peptide bond substitution and the like. Examples of modification of the 2' hydroxyl group of ribose are 2'-O-methylation, 2'-O-methoxyethylation, 2'-O-aminopropyl (AP), 2'-fluoromation, 2'-O-methylcarbamoylethylation, 3,3-dimethylallylation, and deoxyribonucleotidylation and the like. Examples of an intramolecularly cross-linked ribose include nucleotides crosslinked at the 2' and 4' positions (2', 4'-BNA). 2', 4'-BNA includes, for example, locked nucleic acid (α-L-methyleneoxy (4'-CH₂-O-2') BNA or β-D-methyleneoxy (4'-CH₂-O-2') BNA, which is also called LNA, ethyleneoxy (4'-(CH₂)₂-O-2') BNA which is also known as ENA, β-D-thio (4'-CH₂-S-2') BNA, aminooxy (4'-CH₂-O-N(R)-2') BNA (R is H or CH₃), oxyamino (4'-CH₂-N(R)-O-2') BNA (R is H or CH₃) which is also called 2', 4'-BNANC, 2', 4'-BNACOC, 3'-amino-2', 4'-BNA, 5'-methyl BNA, (4'-CH(CH₃)-O-2') BNA which is also called cEt-BNA, (4'-CH(CH₂OCH₃)-O-2') BNA which is also called cMOE-BNA, amide type BNA (4'-C(O)-N(R)-2') BNA (R is H or CH₃) which is called AmNA, and the like. Examples of modification of the base moiety include halogenation; alkylation such as methylation, ethylation, n-propylation, isopropylization, cyclopropylation, n-butylation, isobutylation, s-butylation, t-butylation, cyclobutylation; hydroxylation; amination; deaminolation; demethylation and the like.

### Site-specific editing method for target RNA

A method for site-specific editing of a target RNA comprises contacting the target RNA with a target editing guide RNA, which is an oligonucleotide that induces site-specific editing on the target RNA, in the presence of adenosine deaminase 1. Target editing guide RNA forms a double strand containing a mismatched base with the target RNA, and by specifically recruiting adenosine deaminase 1, the adenosine residue contained in the target RNA can be converted to an inosine residue in a site-specific manner. The site-specific editing method of the target RNA may be performed *in vitro* or *in vivo.*

The site-specific editing method of the target RNA can be performed, for example, by introducing or expressing the above-mentioned target editing guide RNA in eukaryotic cells having the target RNA. In a method for introducing RNA into eukaryotic cells, the target editing guide RNA can be appropriately selected from various methods used in nucleic acid drugs and applied. Further, by introducing a plasmid or the like capable of expressing the target editing guide RNA into the eukaryotic cell, the target editing guide RNA can be expressed in the eukaryotic cells.

By applying a site-specific editing method for target RNA using target editing guide RNA to changes in amino acids involved in the functional expression of intracellular proteins at sites of sugar chain modification, phosphorylation sites, and metal coordination sites, and the like, it will be possible to provide a new methodology for temporarily regulating the functionality of intracellular proteins. In addition, by generalizing the method of regulating the functionality of proteins *in vivo* by a site-specific editing method of target RNA using a target editing guide RNA, it will be a molecular technology that can contribute to the development of research in the field of life science.

Conventionally, nucleic acid drugs using regulation of target protein expression by siRNA or functional control of target protein by functional RNA called aptamer have been developed. However, there is no example of a drug that converts the information of mRNA and modifies the function of the target protein encoded by the mRNA. Therefore, target editing guide RNA has the potential to produce novel nucleic acid drugs with unprecedented efficacy.

For example, a nonsense mutant hereditary disease is a disease caused by the failure to synthesize the original protein due to a stop codon formed by a point mutation on the gene. Nonsense mutant hereditary diseases include, for example, muscular dystrophy, multiple sclerosis, Alzheimer's disease, neurological tissue degeneration, neurological diseases such as Parkinson's disease, and cancer. For example, by editing stop codons such as UAA, UAG, and UGA with a target editing guide RNA, it can be used as a nucleic acid drug having an unprecedented mechanism for the above-mentioned diseases. Specifically, for example, it is conceivable to control protein synthesis by editing UAG, which is a stop codon, into UIG, which is a tryptophan codon.

For example, many proteins that have important functions in cells are precisely controlled on/off by phosphorylation/dephosphorylation, and it has been suggested that abnormalities in phosphorylation/dephosphorylation are deeply involved in various diseases including cancer. Examples of the phosphorylation site of a protein include Tyr, Thr, Ser and the like. By editing the codons encoding these amino acids into codons encoding other amino acids by target editing guide RNA, it becomes possible to suppress the phosphorylation of proteins. Specifically, for example, the phosphorylation of intracellular proteins can be controlled by editing the UAC, which is a Tyr codon, into the UIC, which is a Cys codon.

In another embodiment, the present invention comprises use of a target editing guide RNA in the manufacture of a pharmaceutical composition used in the treatment of hereditary diseases, use of the target editing guide RNA in the treatment of hereditary diseases, and the target editing guide RNA to be used in the treatment of hereditary diseases. The subject of treatment is, for example, a mammal, which includes a human. In addition, the target of treatment may be a non-human animal.

### Examples

Hereinafter, the present invention will be specifically described with reference to the Examples, but the present invention is not limited to these Examples.

### Preparation of adenosine deaminase

Recombinant hADAR2 and hADAR1p110 were prepared as adenosine deaminase by synthesizing and purifying using a yeast expression system, according to the description in the literature ( Macbeth, MR. And Bass, BL. Methods Enzymol. 424, 319-331 (2007) , Fukuda, M. et al. Sci. Rep. Srep41478 (2017) ). In addition, plasmids expressing hADAR2, hADAR1p110 and hADAR1p150 (p YES_A_hADAR2p110, p YES_A_hADAR1p110, p YES_A_hADAR1p150) were prepared by a conventional method.

### Preparation of model target RNA: synthesis of Rluc_sRNA

Rluc_WT RNA (SEQ ID NO: 1) was prepared so as to be 0.2 nM by transcription from the psiCHECK^{™}-2 Vector (Promega) by a conventional method. An *in vitro* editing reaction was performed by adding recombinant hADAR2 to a final concentration of 1 pM, and incubating at 37°C for 2 hours in an editing reaction buffer (20 mM HEPES-KOH (pH 7.5), 2 mM MgCl₂, 100 mM NaCl, 0.5 mM DTT, 0.01% TritonX-100, 5% glycerol).

After the editing reaction, Rluc_WT RNA was purified by phenol/chloroform extraction and ethanol precipitation. Next, the cDNA was synthesized with the Rluc_WT_BamR01 primer (SEQ ID NO: 2) using Primescript Reverse Transcriptase II (TaKaRa). Then, PCR (30 cycles, denaturation: 98°C, 10 seconds; annealing: 55°C, 15 seconds; elongation: 68°C, 60 seconds) was conducted using the Rluc_WT_EcoF01 primer (SEQ ID NO: 3), Rluc_WT_BamR01 primer (SEQ ID NO: 2), and PrimeStar GXL DNA Polymerase (TaKaRa) to amplify the cDNA. Then, the obtained cDNA was cloned into the pUC19 plasmid as the insert DNA as follows. The insert DNA and pUC19 plasmid were digested with EcoR I (TaKaRa) and BamH I (TaKaRa) at 37°C for 1 hour, and then each DNA was purified by phenol/chloroform extraction and ethanol precipitation. After digestion with restriction enzymes, the pUC19 plasmid and the insert DNA were mixed so as to have a molar ratio of 1: 3, and a ligation reaction was carried out using DNA and the Ligation Kit <Mighty Mix> (TaKaRa). The obtained ligation sample was used to transform DH5a, which was then cultured overnight at 37°C using an LB agar plate, and then the plasmid was extracted using the QIAprep Spin Miniprep Kit (QIAGEN). Nucleotide sequence analysis of the obtained plasmid DNA was performed to obtain a sequence in which A122 of Rluc_WT was mutated to G (Rluc_K41R) and a plasmid DNA in which Rluc_K41R was cloned (pUC19-Rluc_K41R).

The 1st PCR (30 cycles, denaturation: 98°C, 10 seconds; annealing: 55°C, 15 seconds; elongation: 68°C, 40 seconds) was performed by the PrimeStar GXL DNA Polymerase (TaKaRa) with pUC19-Rluc_K41R as a template, using the Rluc_NheF01 primer (SEQ ID NO: 4) and the RL_W104X_RV primer (SEQ ID NO: 5) for the 5' side fragment, and the Rluc_XhoR01 primer (SEQ ID NO: 6) and the RL_W104X_FW primer (SEQ ID NO: 7) for the 3' side fragment. Next, each PCR product was diluted 100 fold and 2nd PCR (30 cycles, denaturation: 98°C, 10 seconds; annealing: 55°C, 15 seconds; elongation: 68°C, 60 seconds) was performed by the PrimeStar GXL DNA Polymerase (TaKaRa), using the Rluc_NheF01 primer and Rluc_XhoR01 primer; and DNA (Rluc_K41R_W104X) (SEQ ID NO: 10) having a sequence in which G311 of Rluc_K41R was mutated to A was obtained by purification by phenol/chloroform extraction and ethanol precipitation.

The obtained DNA (Rluc_K41R_W104X) and psiCHECK^{™}-2 Vector (promega) were subjected to restriction enzyme digestion at 37°C for 1 hour using NheI (TaKaRa) and XhoI (TaKaRa), followed by purification of each DNA by phenol/chloroform extraction and ethanol precipitation. Rluc_K41R_W104X and psiCHECK^{™}-2 Vector after digestion with restriction enzymes were mixed so as to have a molar ratio of 3: 1 and a ligation reaction was carried out using the DNA Ligation Kit <Mighty Mix> (TaKaRa). The obtained ligation sample was transformed into DH5a and cultured overnight at 37°C using an LB agar plate. The selected colonies were cultured overnight at 37°C in LB liquid medium, and plasmids were extracted using the QIAprep Spin Miniprep Kit (QIAGEN). Then, the sequence of the plasmid obtained by the nucleotide sequence analysis was confirmed.

The plasmid of which sequence has been confirmed was used as a template, and by PCR (30 cycles, denaturation: 98°C, 10 seconds; annealing: 55°C, 15 seconds; elongation: 68°C, 20 seconds) performed using the T7_Rluc_sRNA_F01 primer (SEQ ID NO: 8) and Rluc_sRNA_R01 primer (SEQ ID NO: 9), and PrimeStar GXL DNA Polymerase (TaKaRa), the template DNA for the *in vitro* transcription reaction was amplified, and purified by phenol/chloroform extraction and ethanol precipitation. Rluc_sRNA (SEQ ID NO: 11) was obtained by *in vitro* transcription using the AmpliScribe T7 Kit (Epicentre Biotechnologies). The synthesized Rluc_sRNA was cut out and purified using a 5% polyacrylamide gel containing 8M urea, and used for the subsequent tests.

The sequences of Rluc_WT_RNA, Rluc_K41R_W104X and Rluc_sRNA, and the sequences of the primers used above are shown below. The underline in the table indicates the adenosine residue of the editing target.

**[Table 1]**

| | **Sequence** | SEQ ID NO |
|---|---|---|
| Rluc_WT_RNA | ACCAUGGCUUCCAAGGUGUACGACCCCGAGCAACGCAAACGCAUGA UCACUGGGCCUCAGUGGUGGCUCGCUGCAAGCAAAUGAACGUGCU GGACUCCUUCAUCAACUACUAUGAUUCCGAGAAGCACGCCGAGAAC GCCGUGAUUUUUCUGCAUGGUAACGCUGCCUCCAGCUACCUGUGGA GGCACGUCGUGCCUCACAUCGAGCCCGUGGCUAGAUGCAUCAUCCC UGAUCUGAUCGGAAUGGGUAAGUCCGGCAAGAGCGGGAAUGGCUCA UAUCGCCUCCUGGAUCACUACAAGUACCUCACCGCUUGGUUCGAGCU GCUGAACCUUCCAAAGAAAAUCAUCUUUGUGGGCCACGACUGGGGG GCUUGUCUGGCCUUUCACUACUCCUACGAGCACCAAGACAAGAUCAA GGCCAUCGUCCAUGCUGAGAGUGUCGUGGACGUGAUCGAGUCCUGG GACGAGUGGCCUGACAUCGAGGAGGAUAUCGCCCUGAUCAAGAGCG AAGAGGGCGAGAAAAUGGUGCUUGAGAAUAACUUCUUCGUCGAGAC CAUGCUCCCAAGCAAGAUCAUGCGGAAACUGGAGCCUGAGGAGUUC GCUGCCUACCUGGAGCCAUUCAAGGAGAAGGGCGAGGUUAGACGGC CUACCCUCUCCUGGCCUCGCGAGAUCCCUCUCGUUAAGGGAGGCAA GCCCGACGUCGUCCAGAUUGUCCGCAACUACAACGCCUACCUUCGG GCCAGCGACGAUCUGCCUAAGAUGUUCAUCGAGUCCGACCCUGGGU UCUUUUCCAACGCUAUUGUCGAGGGAGCUAAGAAGUUCCCUAACACC GAGUUCGUGAAGGUGAAGGGCCUCCACUUCAGCCAGGAGGACGCUC CAGAUGAAAUGGGUAAGUACAUCAAGAGCUUCG | 1 |

**[Table 2]**

| **Primer** | **Sequence** | **SEQ ID NO** |
|---|---|---|
| **Rluc_WT_BamR01** | GAAGGATCCTTACAGGTCCTCCTCTGAGA TCAGCTTCTGCTC | **2** |
| **Rluc_WT_EcoF01** | GCAGAATTCACCATGGCTTCCAAGGTGTA CGAC | **3** |
| **Rluc_NheF01** | GACGCTAGCACCATGGCTTCCAAGGTGTA CGAC | **4** |
| **RL_W104X_RV** | CAGCTCGAACTAAGCGGTGAG | **5** |
| **Rluc_XhoR01** | GAACTCGAGTTACAGGTCCTCCTCTGAGA TCAGCTTCTGCTC | **6** |
| **RL_W104X_FW** | **CTCACCGCTTAGTTCGAGCTG** | **7** |
| **T7_Rluc_sRNA_F01** | CTAATACGACTCACTATAGGGCTGGACTC CTTCATCAAC | **8** |
| **Rluc_sRNA_R01** | **CCAGTCGTGGCCCACAAAG** | **9** |

**[Table 3]**

| | **Sequence** | **SEQ ID NO** |
|---|---|---|
| Rluc_K41R_W104X | ATGGCTTCCAAGGTGTACGACCCCGAGCAACGCAAACGCATGATC ACTGGGCCTCAGTGGTGGGCTCGCTGCAAGCAAATGAACGTGCTG GACTCCTTCATCAACTACTATGATTCCGAGAGGCACGCCGAGAACG CCGTGATTTTTCTGCATGGTAACGCTGCCTCCAGCTACCTGTGGAGG CACGTCGTGCCTCACATCGAGCCCGTGGCTAGATGCATCATCCCTG ATCTGATCGGAATGGGTAAGTCCGGCAAGAGCGGGAATGGCTCAT ATCGCCTCCTGGATCACTACAAGTACCTCACCGCTTAGTTCGAGCTG CTGAACCTTCCAAAGAAAATCATCTTTGTGGGCCACGACTGGGGG CTTGTCTGGCCTTTCACTACTCCTACGAGCACCAAGACAAGATCAAG GCCATCGTCCATGCTGAGAGTGTCGTGGACGTGATCGAGTCCTGGG ACGAGTGGCCTGACATCGAGGAGGATATCGCCCTGATCAAGAGCG AAGAGGGCGAGAAAATGGTGCTTGAGAATAACTTCTTCGTCGAGAC CATGCTCCCAAGCAAGATCATGCGGAAACTGGAGCCTGAGGAGTT CGCTGCCTACCTGGAGCCATTCAAGGAGAAGGGCGAGGTTAGACG GCCTACCCTCTCCTGGCCTCGCGAGATCCCTCTCGTTAAGGGAGGC AAGCCCGACGTCGTCCAGATTGTCCGCAACTACAACGCCTACCTTC GGGCCAGCGACGATCTGCCTAAGATGTTCATCGAGTCCGACCCTG GGTTCTTTTCCAACGCTATTGTCGAGGGAGCTAAGAAGTTCCCTAAC ACCGAGTTCGTGAAGGTGAAGGGCCTCCACTTCAGCCAGGAGGAC GCTCCAGATGAAATGGGTAAGTACATCAAGAGCTTCGTGGAGCGC GTGCTGAAGAACGAGCAGTAA | 10 |
| Rluc_sRNA | GGGCUGGACUCCUUCAUCAACUACUAUGAUUCCGAGAGGCACGC CGAGAACGCCGUGAUUUUUCUGCAUGGUAACGCUGCCUCCAGCU ACCUGUGGAGGCACGUCGUGCCUCACAUCGAGCCCGUGGCUAG AUGCAUCAUCCCUGAUCUGAUCGGAAUGGGUAAGUCCGGCAAGA GCGGGAAUGGCUCAUAUCGCCUCCUGGAUCACUACAAGUACCUC ACCGCUUAGUUCGAGCUGCUGAACCUUCCAAAGAAAAUCAUCUU UGUGGGCCACGACUGG | 11 |

### (Example 1)

As the target editing guide RNA (hereinafter, may be referred to as gRNA) for the model target RNA (Rluc_sRNA), the oligoribonucleotides AD1-gRNA01 (SEQ ID NO: 12), AD1-gRNA03 (SEQ ID NO: 13) and AD1-gRNA04 (SEQ ID NO: 14) having the sequences shown below were prepared by a conventional method.

**[Table4]**

| | **Sequence** | SEQ ID NO |
|---|---|---|
| AD1-gRNA01 | CAGCUCAACCAAGCGGUGAGGUACUUG | 12 |
| AD1-gRNA03 | CAGCUCAACCAAGCGGUGAGGUAC | 13 |
| AD1-gRNA04 | CAGCAGCUCAACCAAGCGGUGAGGUAC | 14 |

### (Reference Example 1)

As the target editing guide RNA for the model target RNA (Rluc_sRNA), 5' AS AD-gRNA (SEQ ID NO: 15; hereinafter, may be abbreviated as 5' AS) and 3' AS AD-gRNA (SEQ ID NO: 16; hereinafter, may be abbreviated as 3' AS) having the sequences shown below were prepared according to the description of International Publication No. 2017/010556. In addition, oligoribonucleotides having the sequence of the antisense region (ASR) in 5' AS and 3' AS (hereinafter, 5' ASR: SEQ ID NO: 17; 3' ASR: SEQ ID NO: 18) were prepared by a conventional method.

**[Table 5]**

| | **Sequence** | **SEQ ID NO** |
|---|---|---|
| 5' AS AD-gRNA | GGUUCAGCAGCUCGAACCAAGGGGUGGA AUAGUAUAACAAUAUGCUAAAUGUUGUU AUAGUAUCCCACCU | 15 |
| 3' AS AD-gRNA | GGGUGGAAUAGUAUAACAAUAUGCUAAA UGUUGUUAUAGUAUCCCACCUAACCAAG CGGUGAGGUACUU | 16 |
| 5' ASR | GGUUCAGCAGCUCGAACCAAG | 17 |
| 3' ASR | GGAACCAAGCGGUGAGGUACUU | 18 |

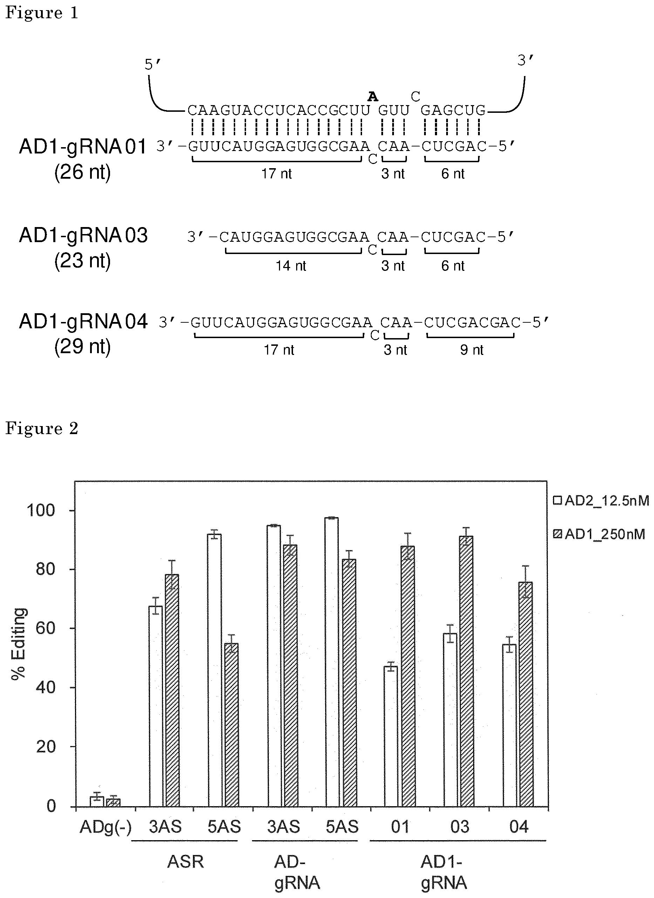

Figure 1 shows a conceptual diagram demonstrating a complex formed from a model target RNA and AD1-gRNA01 and the like. As shown in Figure 1, in the complex, the residue of gRNA corresponding to the adenine residue (A) of the editing target is the cytidine residue (C), forming a mismatched base pair. In addition, there is no residue on the gRNA side that corresponds to the cytidine residue at the 4th residue on the 3' side from the adenine residue on the editing target of the model target RNA, and a mismatched base is inserted into the model target RNA.

### Evaluation of the editing-inducing activity

The editing-inducing activity was evaluated as follows by using the target editing guide RNA prepared above.

### Annealing reaction

0.3 pM Rluc_sRNA and 0.9 pM gRNA of Example 1 and Reference Example 1 were heated in an annealing buffer (10 mM Tri-HCl (pH 7.6), 150 mM NaCl) at 80°C for 3 min and cooled to 25°C in 15 min.

### In vitro editing reaction

Based on the hypothesis that Rluc_sRNA completely forms a complex with the gRNA by the annealing reaction, the following Rluc_sRNA-gRNA complex concentration was calculated. The editing reaction was performed against a 5 nM Rluc_sRNA-gRNA complex in an editing reaction buffer (20 mM HEPES-KOH (pH 7.5), 2 mM MgCl₂, 100 mM NaCl, 0.5 mM DTT, 0.01% TritonX-100, 5% glycerol), by adding 12.5 nM of recombinant hADAR2 or 250 nM of recombinant hADAR1, and then the mixture was incubated at 37°C for 1 hour. The concentrations of hADAR2 and hADAR1 were adjusted to demonstrate the same extent of editing when each was subjected to an editing reaction on the HTR2C RNA (serotonin 2C receptor mRNA precursor) known to be edited by these.

### Editing analysis

After the editing reaction, Rluc_sRNA was purified by phenol/chloroform extraction and ethanol precipitation, and cDNA was synthesized with the Rluc_sRNA_R01 primer (SEQ ID NO: 9) using Primescript Reverse Transcriptase II (TaKaRa). Then, PCR (denaturation: 98°C, 10 seconds; annealing: 55°C, 15 seconds; elongation: 68°C, 20 seconds) was performed using the Rluc_sRNA_F01 primer (SEQ ID NO: 19) and Rluc_sRNA_R01 primer (SEQ ID NO: 9) as well as PrimeStar GXL DNA Polymerase (TaKaRa) to amplify the cDNA. For sequence analysis of the obtained cDNA, a sequencing reaction using the Rluc_sRNA_F01 primer (SEQ ID NO: 19) and the Big Dye Terminator v3.1 Cycle Sequence Kit was performed, and analysis was performed by Applied Biosystem 3500 Genetic Analyzer (Thermo Fisher Scientific). From the chromatographic chart obtained by sequencing, the editing ratio (%) was calculated from the peak height ratio (G/(G + A)) of the target site (A311). The results are shown in Figure 2. Further, from the obtained editing ratio, the ratio of the editing ratio by hADAR1 to the editing ratio by hADAR2 was calculated. The results are shown in Table 7.

**[Table 6]**

| **Primer** | **Sequence** | **SEQ ID NO** |
|---|---|---|
| **Rluc_sRNA_F01** | **GCTGGACTCCTTCATCAAC** | **19** |
| **Rluc_sRNA_R01** | **CCAGTCGTGGCCCACAAAG** | **9** |

**[Table 7]**

| | Ratio of editing ratios hADAR1/hADAR2 |
|---|---|
| ADg (-) | 0. 72 |
| 3' ASR | 1. 16 |
| 5' ASR | 0. 60 |
| 3'AS AD-gRNA | 0. 93 |
| **5' AS** AD-gRNA | 0.86 |
| AD1-gRNA01 | 1. 86 |
| AD1-gRNA03 | 1. 56 |
| AD1-gRNA04 | 1. 39 |

AD1-gRNA showed the same level of editing-inducing activity against hADAR1 as compared with the conventional target editing guide RNA (5' AS, 3' AS). In addition, AD1-gRNA showed an activity of inducing editing specifically for hADAR1.

### (Example 2)

As the target editing guide RNA for the model target RNA (Rluc_sRNA), the oligoribonucleotides AD1-gRNA03.1 (SEQ ID NO: 20), AD1-gRNA03.2 (SEQ ID NO: 21), AD1-gRNA03.3 (SEQ ID NO: 22) and AD1-gRNA03.4 (SEQ ID NO: 23) having the sequences shown below were prepared by a conventional method.

**[Table 8]**

| | **Sequence** | **SEQ ID NO** |
|---|---|---|
| AD1-gRNA03. 1 | CUCAACCAAGCGGUGAGGUAC | 20 |
| AD1-gRNA03. 2 | GCAGCUCAACCAAGCGGUGAGGUAC | 21 |
| AD1-gRNA03. 3 | CAGCAGCUCAACCAAGCGGUGAGGUAC | 22 |
| AD1-gRNA03. 4 | GUUCAGCAGCUCAACCAAGCGGUGAGGUAC | 23 |

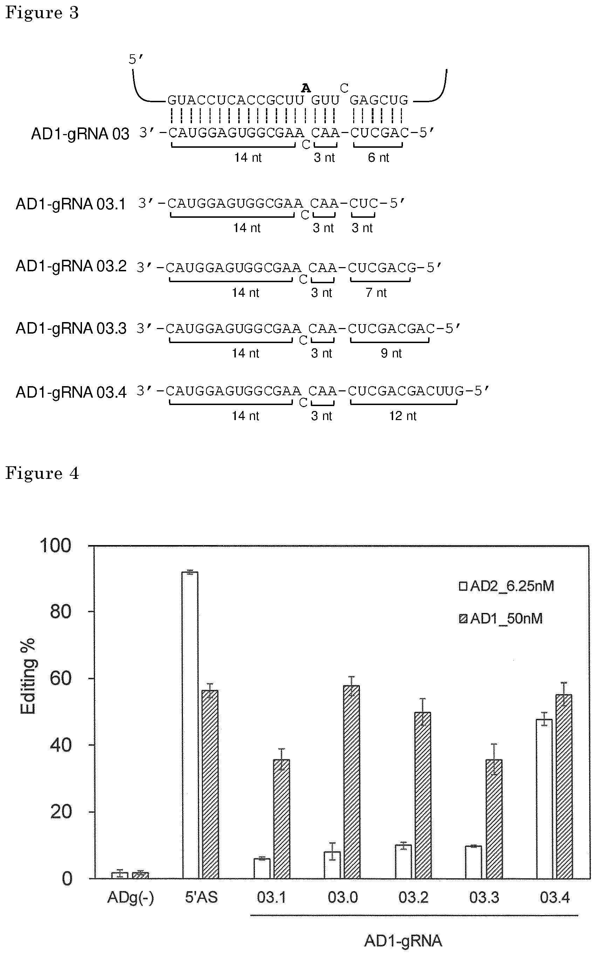

Figure 3 shows a conceptual diagram demonstrating a complex formed from the model target RNA and AD 1-gRNA03 and the like.

### Evaluation of the editing-inducing activity

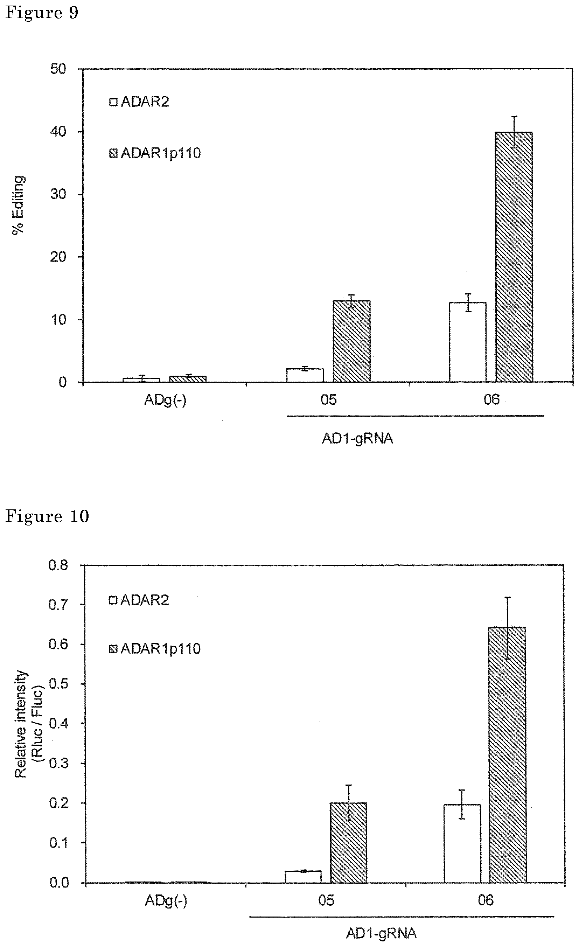

The editing-inducing activity of the obtained oligoribonucleotide was evaluated in the same manner as above. The results are shown in Figures 4 and 9. Since hADAR1 used a different lot from that used above, the concentrations of hADAR1 and hADAR2 used in the editing reaction were different from those of Example 1.

**[Table 9]**

| | Ratio of editing ratios hADAR1/hADAR2 |
|---|---|
| ADg (-) | 1. 05 |
| 5' AS AD-gRNA | 0. 61 |
| AD1-gRNA03.1 | 5. 92 |
| AD1-gRNA03 | 7. 12 |
| AD1-gRNA03. 2 | 4. 97 |
| AD1-gRNA03. 3 | 3.66 |
| AD1-gRNA03. 4 | 1. 15 |

It can be seen that the number of residues of the second oligonucleotide in the target editing guide RNA changes the editing-inducing activity and specificity.

### (Example 3)

A model target RNA in which the mismatched base in the model target RNA was changed to A, U, and G instead of C was prepared in the same manner as above. In addition, the oligoribonucleotide AD1-gRNA03Gm (SEQ ID NO: 24) having the sequence shown below was prepared by a conventional method.

**[Table 10]**

| | **Sequence** | **SEQ ID NO** |
|---|---|---|
| **AD1-gRNA03Gm** | **CAGCUCGAACCAAGCGGUGAGGUAC** | **24** |

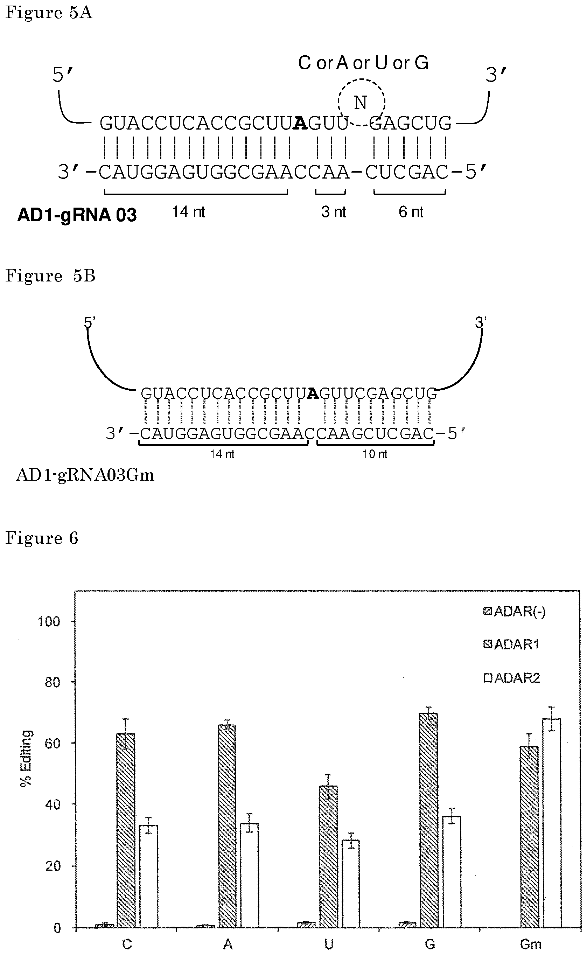

A complex formed from the model target RNA in which the mismatched base is changed and AD1-gRNA03, and a complex formed from the model target RNA and AD1-gRNA03Gm are shown in Figures 5A and 5B. As shown in Figure 5B, in the complex formed from the model target RNA and AD1-gRNA03Gm, there are no mismatched bases other than the target of editing.

### Evaluation of the editing-inducing activity

The editing-inducing activity of AD1-gRNA03 against the model target RNA in which the mismatched base was changed, and the editing-inducing activity of AD1-gRNA03Gm against the model target RNA were evaluated in the same manner as above. The results are shown in Figure 6 and Table 11.

**[Table 11]**

| | Ratio of editing ratios hADAR1/hADAR2 |
|---|---|
| C | 1.90 |
| A | 1. 95 |
| U | 1. 63 |
| G | 1. 93 |
| Gm | 0. 87 |

The type of mismatched base affected the specific editing-inducing activity for hADAR1 to some extent. Further, in AD1-gRNA03Gm in which no mismatched base is present, the specificity for hADAR1 was reduced.

### (Example 4) Evaluation of the editing-inducing activity in cultured cells 1

A plasmid expressing hADAR1p110, pcDNA3.1 (-) Hygro_ADAR1p110, was constructed as follows. In the same manner, the plasmid pcDNA3.1 (-) Hygro_ADAR2 which expresses ADAR2 was constructed.

The 1st PCR was performed with pYES/NT_A_ADAR1p110 as a template, using 0.3 pM of the HisADAR2_XbaF01 primer (SEQ ID NO: 25) and 0.3 pM of the ADAR1-cMyc-1stR01 primer (SEQ ID NO: 26) under the condition of an extension reaction of 3 minutes. By using the 1st PCR product that has been diluted 100 fold as a template, 2nd PCR was performed using 0.3 pM of the HisADAR2_XbaF01 primer (SEQ ID NO: 25) and 0.3 pM of the cMyc_2nd_HindR01 primer (SEQ ID NO: 27) under the condition of an extension reaction of 3 minutes, and the insert DNA was amplified. The insert DNA and pcDNA3.1 (-) Hygro were enzymatically reacted at 37°C for 1 hour using Xba I and Hind III. After purification by phenol/chloroform extraction and ethanol precipitation, transformation was performed by adding 2 pL to 20 pL of E. coli DH5a competent cells (TaKaRa), and culturing the cells overnight on the LB ampicillin agar medium. Using the generated colony as a template, PCR was performed under the condition of an extension reaction of 2 minutes using 0.3 pM of the T7proGGG primer (SEQ ID NO: 28) and 0.3 pM of the BGH reverse primer (SEQ ID NO: 29), insert check was performed, and the clone that produced the amplification product of the fragment length of interest was cultured in liquid. For the sequence of the extracted plasmid, a sequencing reaction was performed by the Big Dye Terminator v3.1 Cycle Sequencing Kit (ABI), using 0.165 pM of the T7proGGG primer, 0.165 pM of the BGH reverse primer, 0.165 pM of the ADAR1_seqF01 primer (SEQ ID NO: 30), 0.165 µM of the ADAR1_seqF02 primer (SEQ ID NO: 31), and 0.165 pM of the ADAR1_seqF03 primer (SEQ ID NO: 32), and a nucleotide sequence analysis was performed.

**[Table 12]**

| | **Sequence** | **SEQ ID NO** |
|---|---|---|
| HisADAR2_XbaF01 | GCTCTAGAACCATGGGGGTTCT CATC | 25 |
| ADAR1-cMyc-1stR01 | CTGAGATCAGCTTCTGCTCTACT GGGCAGAGATAAAAG | 26 |
| cMyc_2nd_HindR01 | CGTAAGCTTCTACAGGTCCTCCT CTGAGATCAGCTTCTGCTC | 27 |
| T7proGGG | CTAATACGACTCCACTATAGGG | 28 |
| BGH reverse | TAGAAGGCACAGTCGAGG | 29 |
| ADAR1_seqF01 | GCAGCACCAGGTGAGTTTCG | 30 |
| ADAR1_seqF02 | GCTCGTGAGATACCTGAACAC | 31 |
| ADAR1_seqF03 | GCTCCGTGTGGAGATGGCGC | 32 |

### (A) Cell culture

HeLa cells were subcultured into a 24-well plate at 5.0 × 10⁴ cells/well and cultured for 48 hours. Transfection was performed using the X-tremeGENE^{™} HP DNA Transfection Reagent (Roche), 50 ng of psiCHECK2^{™} _Rluc_K41R_W104X and 250 ng of pcDNA3.1 (-) Hygro_ADAR1p110 or pcDNA3.1 (-) Hygro_ADAR2, and a plasmid expressing gRNA. As the plasmid expressing gRNA, plasmids expressing 5' AS AD-gRNA, AD1-gRNA01 or AD1-gRNA03 were constructed and used in the same manner as in Reference Example 2 of International Publication No. 2019/11957.

### (B) Editing analysis method

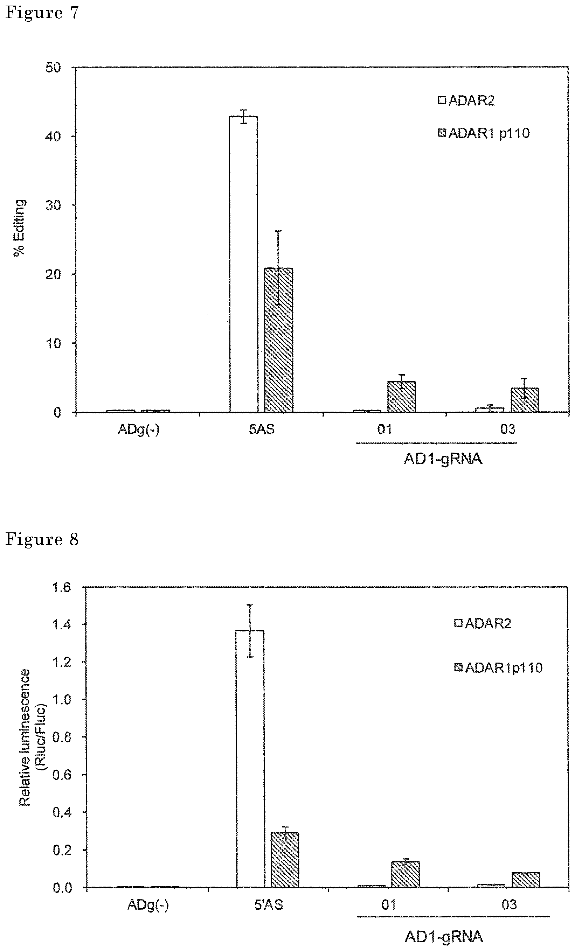

Total RNA was extracted from cells cultured in a 24-well plate using Sepazole RNA I Super G (nacalai tesque), and after DNase treatment was carried out using Recombinant DNase I (TaKaRa), followed by phenol/chloroform extraction and ethanol precipitation. A reverse transcription reaction was performed using PrimeScript II Reverse Transcriptase (TaKaRa), 0.5 pg of total RNA, and 0.25 µM of Oligo (dT) 17 (SEQ ID NO: 33) to amplify cDNA. 1st PCR (30 cycles (denaturation: 98°C, 10 seconds; annealing: 55°C, 15 seconds; elongation: 68°C, 60 seconds) was performed using PrimeStar GXL DNA polymerase (TaKaRa), the Rluc_F01 primer (SEQ ID NO: 34), and the 3'-Adp primer (SEQ ID NO: 35). Using the cDNA obtained by diluting the 1st PCR product 200 fold as a template, 2nd PCR (30 cycles (denaturation: 98°C, 10 seconds; annealing: 55°C, 15 seconds; elongation: 68°C, 60 seconds)) was performed using PrimeStar GXL DNA polymerase (TaKaRa), the Rluc_F01 primer (SEQ ID NO: 34), and the Rluc_R01 primer (SEQ ID NO: 36) to amplify the Rluc fragment. A sequencing reaction was performed using the Big Dye Terminator v3.1 Cycle Sequence Kit (Thermo Fisher Scientific) and 0.165 µM of the Rluc_sRNA_F01 primer (SEQ ID NO: 19), and analysis was performed by Applied Biosystem 3500 Genetic Analyzer (Thermo Fisher Scientific). From the chromatographic chart obtained by sequencing, the editing ratio (%) was calculated from the peak height ratio (G/(G + A)) of the target site (A311). The results are shown in Figure 7 and Table 14.

**[Table 13]**

| | **Sequence** | **SEQ ID NO** |
|---|---|---|
| Oligo(dT)17 | GGCCACGCGTCGACTAGTACTTT TTTTTTTTTTTTTT | 33 |
| Rluc_F01 | CTAATACGACTCACTATAGGGACC ATGGCTTCCAAGGTGTAC | 34 |
| 3'-Adp | GGCCACGCGTCGACTAGTAC | 35 |
| Rluc_R01 | TTACTGCTCGTTCTTCAGCACG | 36 |
| Rluc_sRNA_F01 | GCTGGACTCCTTCATCAAC | 19 |

### (C) Luciferase reporter assay method

A Dual-Luciferase Reporter Assay System (Promega) was used. A cell extract was obtained using 100 pL of Passive Lysis Buffer (Promega) on the cells cultured on a 24-well plate. 100 pL of LARII was added to 20 pL of the obtained cell extract, and 60 seconds later, the emission intensity of Firefly luciferase (Fluc) was measured by GloMax^{(R)} 20/20 Luminometer (Promega). Immediately thereafter, 100 pL of the Stop & Glo Reagent was added, and 60 seconds later, the emission intensity of Renilla luciferase (Rluc) was measured. The emission intensity was normalized by Fluc. The results are shown in Figure 8 and Table 14.

**[Table 14]**

| | **Ratio of editing ratios hADAR1/hADAR2** | |
|---|---|---|
| | **Editing analysis** | **Luciferase reporter assay** |
| ADg (-) | 0. 86 | 1.04 |
| 5' AS AD-gRNA | 0.49 | 0. 21 |
| AD1-gRNA01 | 21. 11 | 14. 51 |
| AD1-gRNA03 | 6. 64 | 6. 04 |

It can be seen that the target editing guide RNA induces RNA editing activity specifically for ADAR1 even inside cells.

### (Example 5) Evaluation of the editing-inducing activity in cultured cells 2

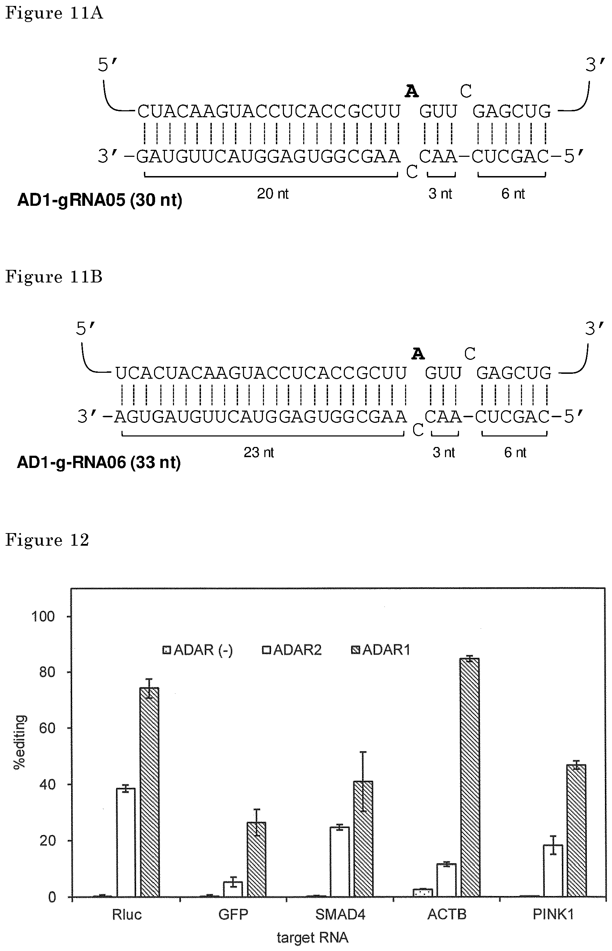

As the plasmid expressing gRNA, plasmids expressing AD 1-gRNA05 (SEQ ID NO: 37) or AD1-gRNA06 (SEQ ID NO: 38) having the sequences shown below were constructed, and used in the same manner as above. The editing-inducing activity in cultured cells was evaluated similarly in Example 4, except for the construction above. The results are shown in Figures 9 and 10. Further, Figure 11A and Figure 11B show conceptual diagrams demonstrating a complex formed from the model target RNA and AD1-gRNA05 or AD1-gRNA06.

**[Table 15]**

| | **Sequence** | **SEQ ID NO** |
|---|---|---|
| AD1-gRNA05 | CAGCTCAACCAAGCGGTGAGGTACTTGTAG | 37 |
| AD1-gRNA06 | CAGCTCAACCAAGCGGTGAGGTACTTGTAGTGA | 38 |

**[Table 16]**

| | **Ratio of editing ratios hADAR1/hADAR2** | |
|---|---|---|
| | **Editing analysis** | **Luciferase reporter assay** |
| ADg (-) | 1. 60 | 0. 15 |
| AD1-gRNA05 | 5. 96 | 6. 89 |
| AD1-gRNA06 | 3. 12 | 3. 26 |

It can be seen that the extension of the first oligonucleotide improves the intracellular editing-inducing activity without impairing the selectivity for ADAR1.

### (Example 6)

AcGFP_sRNA02 (SEQ ID NO: 39) having the sequence shown below was prepared as a model target RNA in the same manner as above. As the target editing guide RNA for the model target RNA, the oligoribonucleotide ADlg_03_GFP_A200 (SEQ ID NO: 40) having the sequence shown below was prepared by a conventional method. The editing-inducing activity of the target editing guide RNA (AD1g_03_GFP_A200) for the model target RNA (AcGFP) was evaluated similarly as in Example 1, except for the changes of the model target RNA and the target editing guide RNA, and the changes in the concentrations of hADAR1 and hADAR2 similarly made in Example 2. The results are shown in Figure 12 and Table 21. In the sequence of the model target RNA, the underlined bold letter indicates the adenosine residue of the editing target, and the underline indicates the mismatched base. The ratio of the editing ratios was calculated from the values obtained by subtracting the editing-inducing activity in the presence of hADAR1 or hADAR2 and the presence of the target editing guide RNA from the editing-inducing activity in the absence of the target editing guide RNA.

**[Table 17]**

| | **Sequence** | **SEQ ID NO** |
|---|---|---|
| AcGFP_sRNA02 | GGGUGAAUGGCCACAAGUUCAGCGUGAGCGGCGAGGGCGAGG GCGAUGCCACCUACGGCAAGCUGACCCUGAAGUUCAUCUGCACC ACCGGCAAGCUGCCUGUGCCCUGGCCCACCCUGGUGACCACCC UGAGCUACGGCGUGCAGUGCUUCUCACGCUA | 39 |
| AD1g_03_GFP_A200 | CUGCACCCGCAGCUCAGGGUGGUC | 40 |

### (Example 7)

PINK1_s02 (SEQ ID NO: 41) showning the sequence below was prepared as a model target RNA in the same manner as above. As a target editing guide RNA for the model target RNA, the oligoribonucleotide AD1g_03_PINK1_A1310 (SEQ ID NO: 42) having the sequence shown below was prepared by a conventional method. The editing-inducing activity of the target editing guide RNA (AD1g_03_PINK1_A1310) for the model target RNA (PINK1_s02) was evaluated in the same manner as in Example 6 except that the model target RNA and the target editing guide RNA were changed. The results are shown in Figure 12 and Table 21.

**[Table 18]**

| | **Sequence** | **SEQ ID NO** |
|---|---|---|
| P!NK1_s02 | GGCUGAUGAGAGCAUCGGCCUGCAGUUGCCCUUCAGCAGCUGG UACGUGGAUCGGGGCGGAAACGGCUGUCUGAUGGCCCAGAGG UGUCCACGGCCCGUCCUGGCCCCAGGGCAGUGAUUGACUACAG CAAGGCUGAUGCCUAGGCAGUGGAGCCAUCGCCUAUGAAAUCU UCGGGCUUGUCAAUCCCUUCUACGGCCAGGGCAAGGCCCACCUU GAAAGC | 41 |
| AD1g_03_PINK1_A1310 | UCCCACGCCCAGGCAUCAGCCUUG | 42 |

### (Example 8)

SMAD_sRNA (SEQ ID NO: 43) having the sequence shown below was prepared as a model target RNA in the same manner as above. The oligoribonucleotide ADlg_03_SMAD4 (SEQ ID NO: 44) having the sequence shown below was prepared by a conventional method as a target editing guide RNA for the model target RNA. The editing-inducing activity of the target editing guide RNA (ADlg_03_SMAD4) for the model target RNA (SMAD_sRNA) was evaluated in the same manner as in Example 6, except that the model target RNA and the target editing guide RNA were changed. The results are shown in Figure 12 and Table 21.

**[Table 19]**

| | **Sequence** | **SEQ ID NO** |
|---|---|---|
| SMAD_sRNA | GGAUGACCUUCGUCGCUUAUGCAUACUCAGGAUGAGUUUUGUGA AAGGCUGGGGACCGGAUUACCCAAGACAGAGCAUCAAAGAAACA CCUUGCUGGAUUGAAAUUCACUUACACCGGGCCCUCCAGCUCCU AGACGAAGUACUUCAUACCAUGCCGAUUGCAGACCCACAACCUUU AGACUG | 43 |
| AD1g_03_SMAD4 | GUACUUGUCCAGGAGCUGGAGGGC | 44 |

### (Example 9)

ACTB_s01 (SEQ ID NO: 45) having the sequence shown below was prepared as a model target RNA in the same manner as above. As a target editing guide RNA for the model target RNA, the oligoribonucleotide ADlg_03_ACTB (SEQ ID NO: 46) having the sequence shown below was prepared by a conventional method. The editing-inducing activity of the target editing guide RNA (AD1g_03_ACTB) for the model target RNA (ACTB_s01) was evaluated in the same manner as in Example 6, except that the model target RNA and the target editing guide RNA were changed. The results are shown in Figure 12 and Table 21.

**[Table 20]**

| | **Sequence** | **SEQ ID NO** |
|---|---|---|
| ACTB_s01 | GGACUUUGAUUGCACAUUGUUGUUUUUUUAAUAGUCAUUCCAAAU AUGAGAUGCGUUGUUACAGGAAGUCCCUUGCCAUCCUAAAAGCC ACCCCACUUCUCUCUAAGGAGAAUGGCCCAGUCCUCUCCCAAGU CCACACAGGGGAGGUGAUAGCAUUGCUUUCGUGUAAAUUAUGUA AUGCAAAAUUUUUUUAAUCUUCGCCUUAAUACUUUUUUAUUUUGUU UUAUUUUGAAUGAUGAGCCUUCGUGCC | 45 |
| AD1g_03_ACTB | AAAGCAUGCCAUCACCUCCCCUGU | 46 |

**[Table 21]**

| | Ratio of editing ratios hADAR1/hADAR2 |
|---|---|
| AD1g_03_GFP_A200 | 5. 18 |
| AD1g_03_PINK1_A1310 | 2. 61 |
| AD1g_03_SMAD4 | 1. 68 |
| AD1g_03_ACTB | 7. 19 |

As shown in the evaluation results of Examples 6 to 9, the target editing guide RNA having a second oligonucleotide with a mismatched base can induce ADAR1-specific editing activity even when the target RNA is changed.

### (Example 10)

As target editing guide RNAs for the model target RNA (Rluc_sRNA) in Example 1, ADlg_03_RL_A287 (SEQ ID NO: 47) and ADlg_03_RL_A287_GG (SEQ ID NO: 48), which are target editing guide RNAs for adenosine residues at positions different from the editing target in Example 1 were prepared by a conventional method.

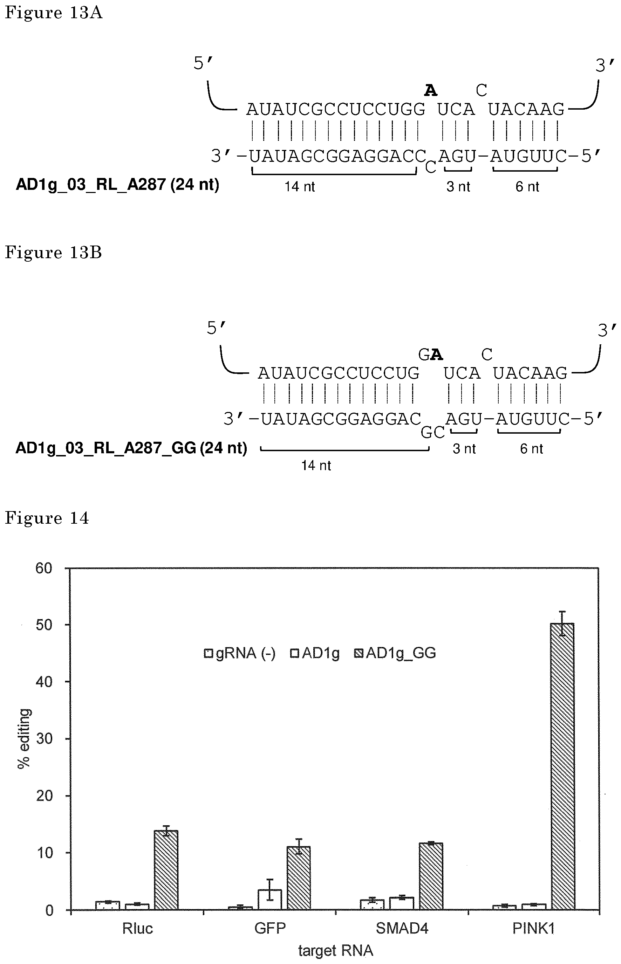

A guanosine residue is bound to the 5' side of the adenosine residue to be edited in the model target RNA, and AD1g_03_RL_A287 has a cytidine residue which is a complementary base to the guanosine residue, and AD1g_03_RL_A287_GG has a guanosine residue which is a non-complementary base. The adenosine residue to be edited in the model target RNA is shown below in bold, and the base sequence forming a complementary strand with the target editing guide RNA is underlined. Figure 13A shows a conceptual diagram of a complex formed from the model target RNA (Rluc_sRNA) and ADlg_03_RL_A287, and Figure 13B shows a conceptual diagram of a complex formed from the model target RNA (Rluc_sRNA) and AD1g_03_RL_A287_GG.

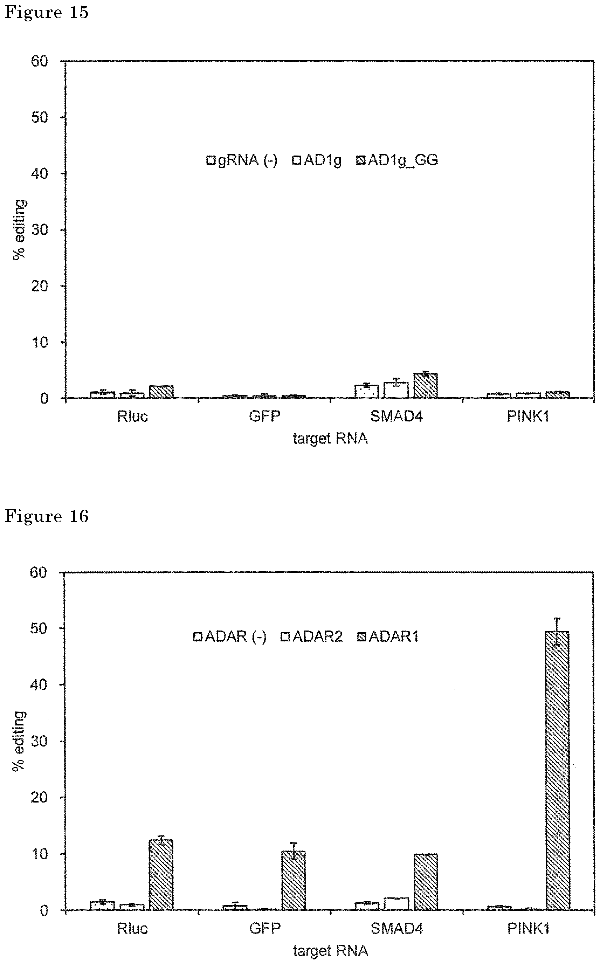

The editing-inducing activity of the target editing guide RNAs (AD1g_03_RL_A287 and ADlg_03_RL_A287_GG) for the model target RNA (Rluc_sRNA) was evaluated in the same manner as in Example 6, except that the model target RNA and the target editing guide RNA were changed. The results are shown in Figures 14 to 16 and Table 26. Figure 14 shows the editing-inducing activity using ADAR1, and Figure 15 shows the editing-inducing activity using ADAR2. Figure 16 and Table 26 show the editing-inducing activities by ADAR1 and ADAR2 and the ratio of the editing ratios when AD1g_03_RL_A287_GG was used as the target editing guide RNA.

**[Table 22]**

| | **Sequence** | **SEQ ID NO** |
|---|---|---|
| Rluc_sRNA | GGGCUGGACUCCUUCAUCAACUACUAUGAUUCCGAGAGGCACGCC GAGAACGCCGUGAUUUUCUGCAUGGUAACGCUGCCUCCAGCUAC CUGUGGAGGCACGUCGUGCCUCACAUCGAGCCCGUGGCUAGAUG CAUCAUCCCUGAUCUGAUCGGAAUGGGUAAGUCCGGCAAGAGCG GGAAUGGCUCAUAUCGCCUCCUGGAUCACUACAAGUACCUCACCG CUUAGUUCGAGCUGCUGAACCUUCCAAAGAAAAUCAUCUUUGUGG GCCACGACUGG | 11 |
| AD1g_03_RL_A287 | CUUGUAUGACCCAGGAGGCGAUAU | 47 |
| AD1g_03_RL_A287_GG | CUUGUAUGACGCAGGAGGCGAUAU | 48 |

### (Example 11)

As target editing guide RNAs for the model target RNA (AcGFP_sRNA02) in Example 6, ADlg_03_GFP_A196 (SEQ ID NO: 49) and AD1g_03_GFP_A196_GG (SEQ ID NO: 50), which are target editing guide RNAs for adenosine residues at positions different from the editing target in Example 6, were prepared by a conventional method. The editing-inducing activity of the target editing guide RNAs (ADlg_03_GFP_A196 and ADlg_03_GFP_A196_GG) for the model target RNA (AcGFP_sRNA02) was carried out in the same manner as in Example 6, except that the model target RNA and the target editing guide RNAs were changed. The evaluation results are shown in Figures 14 to 16 and Table 26.

**[Table 23]**

| | **Sequence** | **SEQ ID NO** |
|---|---|---|
| AcGFP_sRNA02 | GGGUGAAUGGCCACAAGUUCAGCGUGAGCGGCGAGGGCGAGGG CGAUGCCACCUACGGCAAGCUGACCCUGAAGUUCAUCUGCACCAC CGGCAAGCUGCCUGUGCCCUGGCCCACCCUGGUGACCACCCUGA GCUACGGCGUGCAGUGCUUCUCACGCUA | 39 |
| AD1g_03_GFP_A196 | ACGCCGAGCCCAGGGUGGUCACCA | 49 |
| AD1g_03_GFP_A196_GG | ACGCCGAGCCGAGGGUGGUCACCA | 50 |

### (Example 12)

As target editing guide RNAs for the model target RNA (PINK1_s02) in Example 7, AD1g_03_PINK1_A1411 (SEQ ID NO: 51) and AD1g_03_PINK1_A1411_GG (SEQ ID NO: 52), which are target editing guide RNAs for the adenosine residue at a position different from the editing target in Example 7, were prepared by a conventional method. The editing-inducing activity of the target editing guide RNAs (AD1g_03_PINK1_A1411 and AD1g_03_PINK1_A1411_GG) for the model target RNA (PINK1_s02) was evaluated in the same manner as in Example 6 except that the model target RNA and the target editing guide RNA were changed. The results are shown in Figures 14 to 16 and Table 26.

**[Table 24]**

| | **Sequence** | **SEQ ID NO** |
|---|---|---|
| PINK1_s02 | GGCUGAUGAGAGCAUCGGCCUGCAGUUGCCCUUCAGCAGCUGGU ACGUGGAUCGGGGCGGAAACGGCUGUCUGAUGGCCCCAGAGGU GUCCACGGCCCGUCCUGGCCCCAGGGCAGUGAUUGACUACAGCA AGGCUGAUGCCUAGGCAGUGGGAGCCAUCGCCUAUGAAAUCUUC GGGCUUGUCAAUCCCUUCUACGGCCAGGGCAAGGCCCACCUUGA AAGC | 41 |
| AD1g_03_PINK1_A1411 | AGGCGAGGCCCCCACUGCCUAGGC | 51 |
| AD1g_03_PINK1_A1411_GG | AGGCGAGGCCGCCACUGCCUAGGC | 52 |

### (Example 13)

As target editing guide RNAs for the model target RNA (SMAD_sRNA) in Example 8, ADlg_03_SMAD4_A2151 (SEQ ID NO: 53) and ADlg_03_SMAD4_A2151_GG (SEQ ID NO: 54), which are target editing guide RNAs for the adenosine residue at a position different from the editing target in Example 8, were prepared by a conventional method. The editing-inducing activity of the target editing guide RNAs (ADlg_03_SMAD4_A2151 and ADlg_03_SMAD4_A2151_GG) for the model target RNA (SMAD_sRNA) was evaluated in the same manner as in Example 6, except that the model target RNA and the target editing guide RNAs were changed. The results are shown in Figures 14 to 16 and Table 26.

**[Table 25]**

| | **Sequence** | **SEQ ID NO** |
|---|---|---|
| SMAD_sRNA | GGAUGACCUUCGUCGCUUAUGCAUACUCAGGAUGAGUUUGUGAA AGGCUGGGGACCGGAUUACCCAAGACAGAGCAUCAAAGAAACAC CUUGCUGGAUUGAAAUUCACUUACACCGGGCCCUCCAGCUCCUAG ACGAAGUACUUCAUACCAUGCCGAUUGCAGACCCACAACCUUUAG ACUG | 43 |
| AD1g_03_SMAD4_A2151 | AUGAAGACUCCGUCUAGGAGCUGG | 53 |
| AD1g_03_SMAD4_A2151_GG | AUGAAGACUCGGUCUAGGAGCUGG | 54 |

**[Table 26]**

| | Ratio of editing ratios hADAR1/hADAR2 |
|---|---|
| AD1g_03_RL_A287_GG | 12.3 |
| AD1g_03_GFP_A196_GG | 503.8 |
| AD1g_03_PINK1_A1411_GG | 241. 3 |
| AD1g_03_SMAD4_A2151_GG | 4. 7 |

As shown in Figure 16 and Table 26, the target editing guide RNAs having a second oligonucleotide with a mismatched base can induce ADAR1-specific editing activity. Further, as shown in Figures 14 and 15, when the target RNA has a guanosine residue on the 5' side of the editing target base, by introducing a mismatched base G into the target editing guide RNA for the guanosine residue, the editing-inducing activity by ADAR1 is particularly improved.

The disclosure of Japanese Patent Application No. 2019-177118 (Filing Date: September 27, 2019) is incorporated herein by reference in its entirety. All documents, patent applications, and technical standards described herein are incorporated herein by reference to the same extent as if the individual documents, patent applications, and technical standards were specifically and individually stated to be incorporated by reference.

## Claims

1. An oligonucleotide that induces site-specific editing of a target RNA, comprising a first oligonucleotide that identifies the target RNA, and a second oligonucleotide linked to the 5' side of the first oligonucleotide,
wherein the first oligonucleotide consists of:
a target-corresponding nucleotide residue that corresponds to an adenosine residue in the target RNA,
a 10 to 24 residue oligonucleotide linked to the 3' side of the target-corresponding nucleotide residue and having a base sequence complementary to the target RNA, and
a 3 to 6 residue oligonucleotide linked to the 5' side of the target-corresponding nucleotide residue and having a base sequence complementary to the target RNA; and
wherein the second oligonucleotide has 2 to 10 residues and forms a complementary strand with the target RNA, and the complementary strand contains at least one mismatched base.

2. The oligonucleotide of claim 1, wherein the second oligonucleotide has 4 to 8 residues, and the complementary strand contains one mismatched base.

3. The oligonucleotide of claim 1 or 2, wherein the mismatched base is inserted into the complementary strand.

4. The oligonucleotide of any one of claims 1 to 3, wherein the target RNA contains the mismatched base.

5. The oligonucleotide of any one of claims 1 to 4, wherein the site-specific editing is caused by an enzymatic reaction by adenosine deaminase 1.

6. A method for site-specific editing of a target RNA, comprising contacting the oligonucleotide of any one of claims 1 to 5 with the target RNA in the presence of adenosine deaminase 1.

7. The editing method of claim 6, which is performed in eukaryotic cells.
